Europäisches Patentamt

European Patent Office

Office européen des brevets

(19)

(11) Numéro de publication : **0 129 459**
**B1**

(12)  **FASCICULE DE BREVET EUROPÉEN**

(45) Date de publication du fascicule du brevet :
30.12.86

(51) Int. Cl.⁴ : **C 07 C 29/86**

(21) Numéro de dépôt : **84401108.0**

(22) Date de dépôt : **30.05.84**

(54) **Procédé de purification d'un mélange eau-alcool en C1-C2-impuretés, issu d'un procédé de fabrication industriel d'alcool en C1-C2 au moyen d'un agent d'extraction.**

(30) Priorité : **09.06.83 FR 8309562**

(43) Date de publication de la demande :
**27.12.84 Bulletin 84/52**

(45) Mention de la délivrance du brevet :
**30.12.86 Bulletin 86/52**

(84) Etats contractants désignés :
**AT BE CH DE FR GB IT LI LU NL SE**

(56) Documents cités :
**EP-A- 0 008 024**
**FR-A- 2 466 264**
**FR-A- 2 505 616**

(73) Titulaire : **RHONE-POULENC CHIMIE DE BASE**
**25, quai Paul Doumer**
**F-92408 Courbevoie (FR)**

(72) Inventeur : **Ceries, Bernard**
**16, rue Commandant Faurax**
**F-69006 - Lyon (FR)**
Inventeur : **Guidoux, Francois**
**1, avenue de Bellevue**
**F-78150 - Le Chesnay (FR)**

(74) Mandataire : **Rioufrays, Roger et al**
**RHONE-POULENC INTERSERVICES Service Brevets**
**Chimie Centre de Recherches de Saint-Fons B.P. 62**
**F-69192 Saint-Fons Cédex (FR)**

**0 129 459**

## Description

La présente invention concerne un procédé de purification d'un mélange eau-alcool en $C_1$-$C_2$ par un agent d'extraction, ledit mélange provenant d'un procédé de fabrication d'alcool en $C_1$-$C_2$ par synthèse chimique. Par alcool en $C_1$-$C_2$ on entend le méthanol et l'éthanol ou les mélanges de ces deux produits.

Le méthanol peut être fabriqué notamment par oxydation directe par l'oxygène moléculaire d'hydrocarbures, par saponification du chlorure de méthyle ou par hydrogénation du monoxyde de carbone. Au cours de la mise en œuvre de ces procédés et surtout au cours du procédé par oxydation directe, le méthanol se trouve en mélange avec du formaldéhyde, de l'acétaldéhyde, de l'acétone, de petites quantités d'acides, des alcools supérieurs et d'autres aldéhydes et cétones. Il est donc nécessaire d'isoler le méthanol de ces différents produits.

L'éthanol industriel obtenu par synthèse chimique, notamment par hydratation de l'éthylène, contient différentes sortes d'impuretés organiques dissoutes, généralement liquides aux conditions normales, qu'il est nécessaire d'éliminer en vue d'obtenir, entre autres, de l'alcool bon goût.

Ces impuretés peuvent être en particulier des éthers comme le diéthyléther, l'éthylpropyléther, le méthyléthyléther, ... des aldéhydes comme l'acétaldéhyde, des esters comme l'acétate d'éthyle, l'acétate de butyle, ... des cétones comme l'acétone, la méthyléthylcétone, la diéthylcétone, ... des alcools supérieurs en $C_3$-$C_5$ et des produits lourds.

Dans le cas de la synthèse de l'éthanol par hydratation de l'éthylène, les impuretés du type ci-dessus se forment, notamment au cours de l'hydrolyse des sulfates d'éthyle ou de l'hydratation directe de l'éthylène.

On obtient ainsi au cours de procédé un alcool brut plus ou moins concentré, contenant ces impuretés et qu'il est nécessaire de purifier.

Or les méthodes de purification connues de ces alcools en $C_1$-$C_2$, à savoir essentiellement la distillation et l'extraction par solvant, sont lourdes et coûteuses. La purification par distillation est compliquée en raison du fait que l'alcool en $C_1$-$C_2$ dilué à traiter renferme à la fois des impuretés plus volatiles et moins volatiles que l'alcool en $C_1$-$C_2$. L'extraction par solvant est également difficilement envisageable car l'élimination totale du solvant de la phase aqueuse est toujours ardue.

L'utilisation d'un agent d'extraction, à savoir un gaz non toxique, le plus souvent l'anhydride carbonique sous forme d'un liquide à l'état sub-critique ainsi que sous forme d'un fluide à l'état super-critique comme solvant d'extraction est connue depuis longtemps.

On a ainsi utilisé $CO_2$ pour extraire notamment des aromes (brevet des Etats-Unis d'Amérique n° 3 477 856), des aromes de vin (demande de brevet français 2 505 616), des graisses de grains (brevet des Etats-Unis d'Amérique n° 3 939 281), de la caféine du café (brevet des Etats-Unis d'Amérique n° 3 843 832).

Une étude très complète de l'extraction par ces gaz à l'état super-critique a été publiée dans l'édition internationale en anglais de la revue Angwandte Chemie, N° 17 : 10, pages 701-784 (octobre 1978).

Par ailleurs dans le brevet français 2 466 264 on décrit un procédé et un appareillage d'extraction de l'éthanol à partir d'un mélange eau-éthanol par du $CO_2$ liquide.

Cependant, la présente invention a permis de mettre en évidence de façon surprenante et inattendue qu'il est possible d'extraire de manière simple et économique la plupart des impuretés dissoutes dans un mélange eau-alcool en $C_1$-$C_2$ issu d'un procédé de fabrication d'alcool en $C_1$-$C_2$ industriel par synthèse chimique au moyen d'un gaz, de préférence le $CO_2$, à l'état liquide ou super-critique sans extraire de ce mélange des quantités trop importantes d'alcool en $C_1$-$C_2$.

La présente invention concerne en effet un procédé de purification d'un mélange eau-alcool en $C_1$-$C_2$ issu d'un procédé de fabrication industriel d'alcool en $C_1$-$C_2$ par synthèse chimique, caractérisé en ce qu'on met en contact au moins une fois le mélange à traiter avec au moins un gaz non toxique à l'état liquide ou super-critique et on sépare l'extrait obtenu contenant les impuretés du raffinat purifié.

Comme gaz non toxique on utilise de préférence $CO_2$ que l'on peut remplacer en tout ou partie par un gaz non toxique choisi parmi $N_2O$, $SF_6$, $CF_3Cl$, $CHF_2Cl$, $CH_2 = CF_2$, $C_3F_8$, $CHF_3$, $C_2H_6$, $C_2H_4$, etc...

Dans certains procédés de préparation de l'éthanol et dans la plupart des procédés de préparation du méthanol, ces alcools se trouvent dans des mélanges liquides exempts d'eau. Pour appliquer le procédé de l'invention, on ajoute à ces mélanges de l'eau en quantités appropriées.

Dans le cas du méthanol, la teneur en eau du mélange à purifier n'est pas critique et peut être située entre 5 et 95 % en poids, de préférence entre 10 et 50 %.

La teneur en éthanol du mélange eau-éthanol peut varier de 5 % en poids environ jusqu'à la teneur de l'azéotrope du mélange eau-éthanol (environ 95 % en poids).

Le procédé est particulièrement intéressant pour des teneurs en alcool en $C_1$-$C_2$ comprises entre 10 et 35 % en poids.

Ainsi la teneur en éthanol des mélanges à traiter provenant du procédé par hydratation de l'éthylène est comprise généralement entre 20 et 70 % en poids et contient entre 0,5 et 2 % en poids d'impuretés citées ci-dessus et le procédé de l'invention est avantageusement applicable à ces mélanges.

Il n'était pas du tout prévisible que la plupart de ces impuretés présente dans le type de mélange à purifier des rapports de partage plus favorables à l'agent d'extraction, en particulier au $CO_2$, qu'au

mélange eau-alcool en $C_1$-$C_2$. De plus ces impuretés sont généralement moins polaires que l'alcool en $C_1$-$C_2$ et présentent une constante diélectrique $\epsilon$ plus faible que celle de l'alcool en $C_1$-$C_2$ aux conditions de température et de pression de l'étape de mise en contact. On utilise de préférence du $CO_2$ liquide ou super-critique comme fluide d'extraction.

Dans le cas de l'utilisation de $CO_2$ liquide on peut opérer en continu ou en discontinu à une température comprise entre 0 et 31 °C et à une pression comprise entre 35 et 300 bars, de préférence entre 10 et 31 °C, à une pression comprise entre environ 45 et 150 bars, par exemple une pression de 65 bars à 20 °C.

On peut également utiliser du $CO_2$ super-critique. Dans ce cas il est nécessaire que la température soit supérieure à 31 °C, de préférence supérieure à 35 °C pour une pression comprise entre 73 et 350 bars, de préférence entre 100 et 250 bars.

Le procédé selon l'invention peut être mis en œuvre en continu ou en discontinu.

En continu, on utilise un rapport du débit massique de $CO_2$ liquide ou super-critique au débit massique du mélange à traiter compris entre 1 et 100, de préférence entre 2 et 10.

En discontinu, on utilise entre 1 et 100 kg de $CO_2$ par kg de mélange, de préférence entre 2 et 10 kgs.

La quantité optimum de $CO_2$ à utiliser est essentiellement fonction de la teneur en impuretés, du titre en alcool en $C_1$-$C_2$ du mélange à traiter et des pertes en alcool en $C_1$-$C_2$ entraîné par le $CO_2$ que l'on estime admissibles.

Le $CO_2$ se charge d'un extrait riche en éthanol dans lequel les impuretés se sont considérablement concentrées et le mélange résiduaire appelé raffinat ne renferme par contre pratiquement plus d'impuretés, tout en étant légèrement appauvri en alcool en $C_1$-$C_2$.

La mise en contact du mélange à traiter par $CO_2$ liquide ou super-critique se fait dans un appareillage d'extraction liquide-liquide conventionnel sous pression fonctionnant en continu ou en discontinu.

Dans le procédé en discontinu, on mélange dans un réacteur sous pression muni de préférence d'un moyen d'agitation, par exemple dans un autoclave muni d'un agitateur, le mélange à purifier avec $CO_2$ liquide ou super-critique. $CO_2$ se charge en impuretés avec un peu d'alcool en $C_1$-$C_2$. Il est également possible et même avantageux d'opérer en continu. On introduit dans ce cas en continu dans un réacteur sous pression muni éventuellement d'un moyen de chauffage et d'agitation $CO_2$ liquide ou super-critique et le mélange à traiter.

De façon à obtenir une meilleure division du liquide on peut utiliser notamment des colonnes pulsées, des colonnes à garnissage, des mélangeurs décanteurs, des colonnes à plateaux, des chicanes, des systèmes de type Rashig, des filtres microporeux, des matières frittées, sous réserve que l'appareillage utilisé résiste à la pression d'utilisation.

On peut également pulvériser le mélange à traiter dans le $CO_2$ au moyen d'une buse ou tout autre système. Il est avantageux d'effectuer l'extraction à contre-courant. Dans ce cas on utilise comme réacteur une colonne d'extraction. Il est avantageux d'injecter le mélange à purifier en tête de colonne d'extraction, d'alimenter la colonne en $CO_2$ liquide ou super-critique en pied de colonne, de soutirer en continu le mélange purifié en pied de colonne, d'évacuer le $CO_2$ contenant les impuretés et un peu d'alcool en $C_1$-$C_2$ en tête de colonne, de fractionner cette même phase dans un dispositif sous pression, de récupérer les impuretés sous la forme d'un extrait eau-alcool en $C_1$-$C_2$ et de recycler le $CO_2$ vers la colonne d'extraction après l'avoir ramené aux conditions d'extraction. Les impuretés qui se seront concentrées dans l'extrait (mélange eau-alcool en $C_1$-$C_2$ riche en éthanol) peuvent être de natures très variées. En effet par exemple dans le cas de l'éthanol, elles peuvent être plus volatiles que l'éthanol comme, par exemple, le diéthyléther et l'acétaldéhyde ; elles peuvent être aussi volatiles que l'éthanol (méthyléthylcétone) ou moins que l'éthanol (diéthylcétone, n-butanol, butanol-2 ...). Toutefois de façon générale, de meilleurs résultats sont obtenus lorsque les impuretés considérées sont des alcools supérieurs, des éthers, des cétones, des aldéhydes, des esters ou des mélanges de ces types de composés, et, de façon générale, des produits apolaires.

Par la mise en œuvre du présent procédé, on obtient un rapport de concentration des impuretés de l'ordre de 3 à 10, et, dans certains cas ce rapport peut être supérieur à 10. Ce rapport de concentration des impuretés (K) est donné par la relation :

$$K = I_1 : I_2$$

dans laquelle $I_1$ représente le rapport massique (impuretés/éthanol) dans l'extrait que contient le $CO_2$ et $I_2$ le rapport massique (impuretés/alcool en $C_1$-$C_2$) dans la phase eau-alcool en $C_1$-$C_2$ en équilibre avec la phase $CO_2$.

La séparation du $CO_2$ liquide ou super-critique et des impuretés dont il s'est chargé peut être réalisé par différents moyens physiques tels que : élévation de température, détente ou emploi de matière absorbante dans le circuit d'extraction. Dans le cas du $CO_2$ liquide, on peut utiliser une simple distillation.

Les impuretés concentrées sous la forme d'un extrait dégazé eau-alcool en $C_1$-$C_2$ riche en impuretés, peut être, suivant un mode de mise en œuvre avantageux du procédé de l'invention, ultérieurement fractionné par exemple en effectuant sur cet extrait une opération de relargage par dilution à l'eau suivie d'une décantation, on recycle la phase aqueuse contenant l'alcool en $C_1$-$C_2$ à l'étape d'extraction et on élimine la phase organique contenant les impuretés. En effectuant cette opération de relargage on évite pratiquement toute perte d'alcool en $C_1$-$C_2$.

La récupération de l'extrait riche en impuretés contenu dans le $CO_2$ liquide ou super-critique peut s'effectuer par simple distillation du $CO_2$ à une pression inférieure à la pression critique. Une détente est donc nécessaire si l'on travaille avec du $CO_2$ super-critique (pression supérieure à la pression critique ; température supérieure à la température critique). Cette distillation nécessite peu d'étages théoriques car le $CO_2$ est bien plus volatil que l'eau, l'alcool en $C_1$-$C_2$ ou que n'importe laquelle des impuretés. Elle ne consomme que peu d'énergie si l'on est au voisinage du point critique : par exemple, la chaleur de vaporisation du $CO_2$ liquide n'est que de 29 Kcal/kg de $CO_2$ à 65 bars et 23 °C. Cette distillation s'effectue à basse température (au voisinage de 15-25 °C suivant la pression) et permet donc d'utiliser des sources de calories de bas niveau thermique (peu coûteuses). Le $CO_2$ gazeux récupéré en tête de colonne de distillation est condensé puis il peut être recyclé à la colonne de purification.

Selon un mode de mise en œuvre encore plus préféré :

— On met en contact un mélange eau-alcool en $C_1$-$C_2$-impuretés au moins une fois avec du $CO_2$ liquide en vue de former d'une part une phase $CO_2$ liquide contenant la plus grande partie des impuretés et un peu d'alcool en $C_1$-$C_2$ et, d'autre part un raffinat purifié contenant la presque totalité de l'alcool en $C_1$-$C_2$ engagé et de faibles quantités de $CO_2$.

— On sépare la phase $CO_2$ liquide du raffinat purifié.

— On récupère le raffinat ainsi produit et on le soumet à une opération de dégazage permettant d'éliminer le $CO_2$.

— On distille la phase $CO_2$ liquide pour obtenir une phase gazeuse de $CO_2$ purifié et une phase liquide constituée essentiellement des impuretés et d'un peu d'alcool en $C_1$-$C_2$, appelée extrait eau-alcool en $C_1$-$C_2$ riche en impuretés.

— On condense la phase gazeuse $CO_2$ que l'on recycle à l'étape initiale de mise en contact.

— On récupère éventuellement les impuretés en effectuant sur la phase liquide constituée des impuretés et d'un peu d'alcool en $C_1$-$C_2$ l'opération de relargage décrite ci-dessus avec recyclage de la phase aqueuse contenant l'alcool en $C_1$-$C_2$ à l'étape de mise en contact.

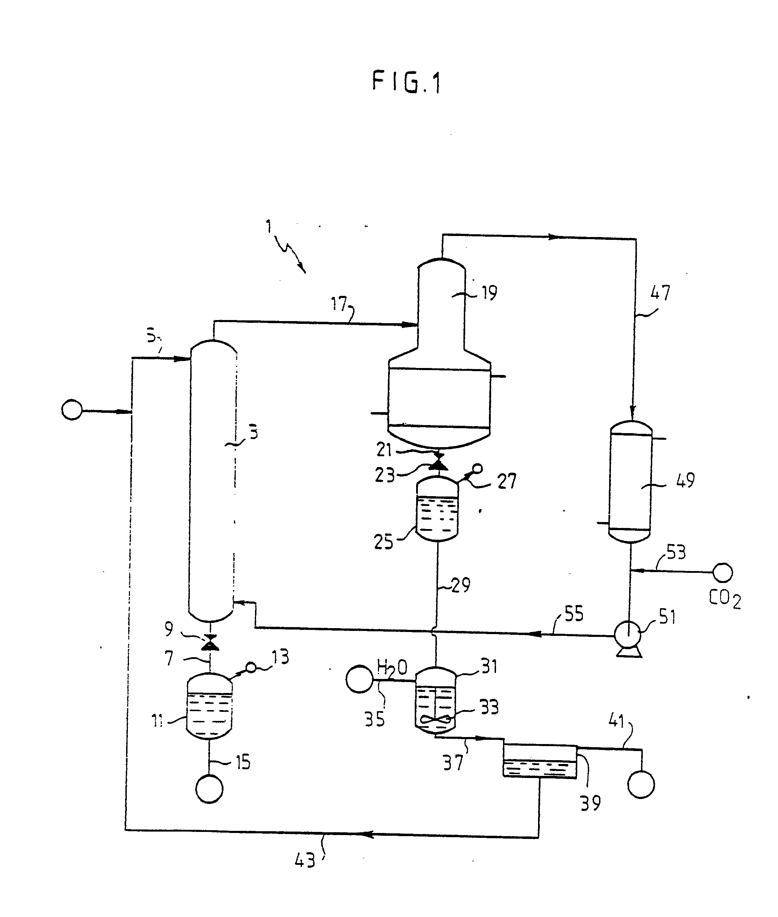

Le procédé préféré décrit ci-dessus peut être mis en œuvre industriellement dans l'installation représentée schématiquement sur la figure 1 du dessin annexé.

Sur cette figure on voit que l'appareillage 1 est essentiellement constitué d'une colonne à plateaux 3 alimentée en tête par la canalisation 5 de mélange eau-alcool en $C_1$-$C_2$ à purifier et en pied par du $CO_2$ liquide. La purification s'effectue et le mélange eau-alcool en $C_1$-$C_2$ purifié est soutiré en continu en pied de colonne par la canalisation 7 munie d'une vanne 9, cette canalisation aboutissant à un dégazeur 11 muni d'une sortie de $CO_2$ 13 et d'une vidange 15 de mélange eau-alcool en $C_1$-$C_2$ purifié. Le $CO_2$ liquide chargé en impuretés est évacué en continu en tête de colonne et s'écoule au moyen de la canalisation 17 dans un autoclave sous pression 19 dans lequel on effectue une distillation.

L'extrait contenant des impuretés en solution dans un peu d'alcool en $C_1$-$C_2$ est soutiré en bas de l'autoclave au moyen de la canalisation 21 munie de la vanne 23 et aboutit dans un dégazeur 25 muni d'une évacuation 27 de $CO_2$ gazeux. L'extrait est introduit par la canalisation 29 dans un récipient 31 muni d'un agitateur 33 et d'une arrivée d'eau 35 en vue d'effectuer l'opération de relargage. Le mélange obtenu est dirigé par la conduite 37 dans un bassin de décantation 39. La couche organique contenant les impuretés est éliminée par la conduite 41 et la couche aqueuse contenant de l'alcool en $C_1$-$C_2$ est recyclée par la conduite 43 en tête de colonne. Le $CO_2$ gazeux est évacué de l'autoclave 19 par la canalisation 47, passe dans un condenseur 49 et est recyclé au moyen du circulateur 51 et, après appoint de $CO_2$ par la conduite 53, en pied de colonne par la canalisation 55.

Les avantages du procédé selon l'invention sont nombreux. Parmi ces avantages on peut citer l'obtention d'un très bon coefficient de transfert entre les phases $CO_2$ et le mélange à purifier aboutissant à un encombrement réduit des appareillages, à l'obtention d'une séparation aisée de $CO_2$ et des impuretés, le $CO_2$ étant bien plus volatil que les impuretés qu'il dissout. De plus la mise en œuvre du procédé ne nécessite qu'une faible dépense énergétique au niveau de la séparation $CO_2$-impuretés. Enfin les coefficients de partage K sont élevés et les gaz non toxiques et en particulier le $CO_2$ utilisé ne laissent aucune trace dans l'alcool en $C_1$-$C_2$.

D'autres avantages et caractéristiques de la présente invention apparaîtront à la lecture des exemples suivants donnés à titre illustratif, nullement limitatif : référence sera faite au dessin annexé sur lequel :

— la figure 2 est un schéma d'un appareillage pour la mise en œuvre en discontinu du procédé selon l'invention,

— la figure 3 est un schéma d'un appareillage pour la mise en œuvre en continu du procédé selon l'invention.

Exemple 1 — Mise en œuvre en discontinu sur un mélange eau-éthanol-impuretés.

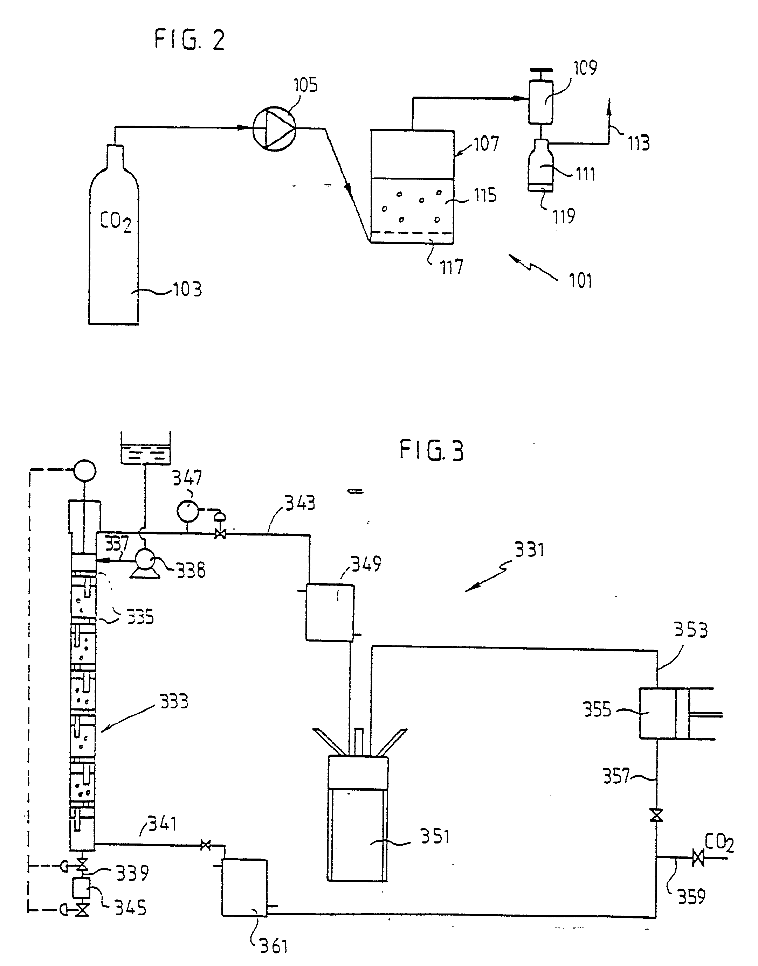

On utilise dans cet exemple l'appareillage représenté schématiquement sur la figure 2. Sur cette figure on voit que l'appareillage 101 est essentiellement constitué d'une source 103 de $CO_2$ à 50 bars d'un compresseur 105 alimentant en $CO_2$ un autoclave 107 muni d'une vanne de détente 109 reliée à un flacon 111 muni d'une évacuation 113. Le mélange 115 eau-éthanol-impuretés à purifier est chargé dans l'autoclave 107 qui est mis sous pression de $CO_2$ à partir de la source 103 (50 bars ; 15 °C). Le compresseur 105 permet de monter la pression aux environs de 95 bars, la température est environ de 23 °C dans l'autoclave.

On ouvre alors la vanne 109 de détente en aval de l'autoclave 107 tout en maintenant la pression constante dans l'autoclave 107 à l'aide du compresseur 105, le $CO_2$ liquide, dispersé par une plaque 117 en métal fritté traverse le mélange 115 chargé dans l'autoclave, se charge en extrait puis est détendu au moyen de la vanne 109 de détente de 95 à 1 bar. L'extrait 119 est récupéré dans le flacon 111. Quand on a fait circuler une masse donnée de $CO_2$, on ramène l'autoclave à pression atmosphérique ; on effectue un prélèvement du résidu ou raffinat contenu dans l'autoclave. L'ensemble de l'opération est recommencé autant de fois que nécessaire pour fractionner les extraits et on obtient ainsi une succession d'extraits et de résidus numérotés qui sont analysés par chromatographie en phase gazeuse.

On charge 48 g dans l'autoclave d'un mélange (M) eau-éthanol à purifier dont la composition est donnée dans le tableau 1 ci-après. On fait circuler à travers le mélange chargé du $CO_2$ liquide (95 bars ; 22 °C) en trois fractions successives (155 g, 155 g et 200 g). Chaque fraction i (i = 1, 2, 3) dissout un extrait Ei et on obtient un raffinat Ri. L'extrait Ei est récupéré par détente du $CO_2$. On analyse en chromatographie les extraits Ei et les raffinats Ri successifs.

Les résultats sont rassemblés dans le tableau 1 ci-après. Ils sont exprimés en ppm pour les impuretés et en % masse pour l'éthanol.

Les coefficients de partage K de différentes impuretés sont indiqués dans le tableau 2 ci-après.

Dans les tableaux les conventions suivantes sont utilisées :

| | |
|---|---|
| MEC | désigne la méthyléthylcétone |
| AcOEt | désigne l'acétate d'éthyle |
| i-PrOH | désigne l'isopropanol |
| EtOH | désigne l'éthanol |
| DEC | désigne la diéthylcétone |
| M2B2 | désigne le méthyl-2 butanol-2 |
| s-BuOH | désigne le butanol secondaire |
| n-PrOH | désigne le n-propanol |
| i-BuOH | désigne l'isobutanol |
| P3 | désigne le pentanol-3 |
| P2 | désigne le pentanol 2 |
| n-BuOH | désigne le n-butanol |
| INI | impureté non identifiée. |

Des tableaux 1 et 2 il ressort que le coefficient de concentration pour l'ensemble des impuretés reste voisin de 10 au cours de l'opération de purification.

(Voir Tableaux 1 et 2 page 6)

Exemple 2 — Mise en œuvre en continu sur un mélange eau-éthanol-impuretés.

On utilise dans cet exemple l'appareillage représenté schématiquement sur la figure 3. Sur cette figure on voit que l'appareillage 331 est constitué d'une colonne 333 à plateaux 335 perforés. Elle comporte 16 étages à raison de 75 mm par étage. Le $CO_2$ liquide constitue la phase dispersée, le mélange à purifier la phase continue. Le mélange à purifier est introduit en tête de colonne par la canalisation 337 à l'aide d'une pompe à membrane 338 suivant un débit compris entre 0 et 700 $cm^3$/h puis est soutiré en pied de colonne par la canalisation 339.

Le $CO_2$ liquide ou super-critique est alimenté en pied de colonne par la canalisation 341 (0 à 20 000 $cm^3$/h) et est soutiré en tête par la canalisation 343. On régule la pression en $CO_2$ de la colonne au moyen d'un système de régulation 347 et le niveau du mélange par une régulation de niveau. Le soutirage du mélange en pied de colonne du mélange purifié s'effectue au moyen d'un sas 345 de 2 $cm^3$ commandé par la régulation de niveau. La canalisation 343 conduit l'extrait du système de régulation 347, à un vaporisateur 349, puis dans un autoclave 351 de 4 litres dans lequel on termine l'évaporation du $CO_2$ et la récupération de l'extrait. Le $CO_2$ gazeux distillé est dirigé par la canalisation 353 vers le compresseur 355. 8N $m^3$/h de $CO_2$ dont le débit est régulé.

Le $CO_2$ liquide est conduit par une canalisation 357, à laquelle aboutit un tube 359 donnant l'appoint en $CO_2$ à 50 bars, à un refroidisseur 361 qui amène le $CO_2$ liquide à 20 °C avant de l'introduire dans la canalisation 341.

L'ensemble des canalisations sont en tube inox de 2,4 mm de diamètre. La pression maximale que peut supporter l'appareillage est de 250 bars.

On a réalisé les 10 essais suivants à la température ambiante :

— Essais C22 et C23 avec alimentation en mélange eau-éthanol-impuretés titrant environ 27 % en

Tableau 1 : Analyses

| | MEC | AcDET | i-PrOH | EtOH | DEC | M2B2 | s-BUOH | n-PrOH | i-BuOH | PE | P2 | n-BuOH | lourds | Total |
|---|---|---|---|---|---|---|---|---|---|---|---|---|---|---|
| M | 1133 | 20 | 88 | 26,6 | 287 | 753 | 1598 | 20 | 153 | 29 | 29 | ND | 331 | 5150 |
| R1 | 418 | 14 | 33 | 23,8 | 62 | 175 | 808 | 23 | 75 | | 9 | ND | 49 | 228 |
| R2 | 113 | 7 | 31 | 21,45 | 8 | 36 | 397 | 16 | 70 | | 4 | ND | 11 | 935 |
| R3 | 19 | 0 | 21 | 16,6 | <5 | 0 | 129 | 11 | 63 | | 3 | ND | 0 | 31 |
| E1 | 20400 | 2302 | 2867 | 63,25 | 9374 | 21600 | 15500 | 1593 | 402 | 188 | 1475 | ND | 9966 | 9920 |
| E2 | 6396 | 981 | 624 | 67,4 | 2528 | 6316 | 12100 | 396 | 159 | 13 | 394 | ND | 3802 | 38200 |
| E3 | 1539 | 337 | 107 | 68,7 | 242 | 741 | 5202 | 39 | 138 | 2 | 36 | ND | 708 | 10700 |

Tableau 2 : Coefficients de partage K des impuretés

| OPERATION n° | MEC | DEC | M2B2 | s-BUOH | Acétone | AcOEt | TOTAL IMPURETES |
|---|---|---|---|---|---|---|---|
| 1 | 10,5 | 21,4 | 18,5 | 5,1 | 9,4 | 52 | 10,6 |
| 2 | 8,1 | 24,2 | 20,1 | 6,7 | 30 | 30 | 7,96 |
| 3 | 6,45 | 10,3 | 9,93 | 6,3 | 26,7 | 26,7 | 5 |

0 129 459

poids d'éthanol.

— Essais C24-C25-C26 et C27 avec alimentation en mélange des essais C22 et C23 mais dilués deux fois.

— Essais C28-C29-C30 et C31 avec alimentation en mélange des essais C22 et C23 mais dilués quatre fois.

Le tableau 3 ci-après définit les conditions opératoires de chaque essai : pression de la colonne d'extraction, pression de l'autoclave de distillation, débits de $CO_2$ liquide, d'alimentation du mélange à purifier, de soutirage du mélange purifié, de soutirage de l'autoclave de distillation, teneurs en éthanol des alimentations, des soutirages et des extraits. La dernière colonne du tableau 3 définit le taux de rétention de phase dispersée ($CO_2$ liquide) dans la colonne.

La durée de chaque essai est au minimum égale à 2 heures : on renouvelle au moins 3 fois le volume de la colonne avant de considérer la marche de l'installation comme stable. On notera que les valeurs de teneurs en éthanol des soutirages de colonnes indiquées au tableau 3, sont proches l'une de l'autre révélant ainsi une bonne stabilité de marche. Pour les forts débits en $CO_2$ des problèmes d'engorgement de colonne apparaissent.

Essai C22 et C23

On alimente la tête de colonne en mélange à purifier suivant un débit de 630 cm³/h et on soutire en pied de colonne suivant un débit de 580 cm³/h. Compte tenu du volume des tubes de raccordement, on ne récupère qu'environ 35-40 cm³/h au pied de l'autoclave de distillation au lieu de 50 cm³/h théoriques.

Quand le régime permanent est atteint on analyse par chromatographie en phase gazeuse les impuretés dont la formule chimique est identifiée, du mélange alimenté et des différents soutirages : soutirage du pied de colonne (résidu), soutirage du pied de l'autoclave de distillation (extrait).

Les résultats sont rassemblés au Tableau 4 ci-après. Les facteurs des pics sont égaux à 1. Les colonnes du tableau 4 sont numérotées et les commentaires ci-après utilisent ces numéros.

Les essais C22 et C23 sont mis en œuvre avec un débit de mélange à purifier de 630 cm³/h et deux débits de $CO_2$ liquide, respectivement 3, 6 et 2 kg/h. Si l'on compare la colonne 3 et la colonne 4, on constate que l'opération de purification est efficace car la plupart des impuretés sont totalement ou presque totalement éliminées. La colonne 5 comparée à la colonne 4 montre qu'en diminuant le débit de $CO_2$ de 3,6 à 2 kg/h, la purification est moins efficace. Les colonnes 6 et 2 reprennent les teneurs en impuretés des soutirages autoclaves. Ces teneurs sont très élevées et les facteurs de concentrations obtenus entre 6 et 3 sont supérieurs à 10.

Essais C24-C25-C26-C27

La mise en œuvre est identique à celles des essais C22 et C23 et les résultats sont rassemblés dans le tableau 4, les colonnes 8 et 9 représentant les analyses des alimentations en mélange à purifier de la colonne. Toutefois les essais C24-C25 ont été effectués avec un débit de mélange à purifier de 620 cm³/h et deux débits de $CO_2$ liquide de 4,5 et 6 kg/h. Par contre les essais C26 et C27 ont été effectués avec un débit de mélange à purifier de 250 cm³/h et deux débits de $CO_2$ liquide de 4,3 et 7,4 kg/h.

Il ressort du tableau 4 que la purification est plus efficace quand le rapport de débit $CO_2$/débit mélange à purifier est plus élevé. Si on compare les essais C22 et C24 (colonnes 4 et 10), on constate que pour des débits de $CO_2$ liquide et de mélange à purifier comparables, l'efficacité de la purification est similaire pour des pertes en éthanol comparables (diminution de la teneur pondérale en éthanol de 24,6 % à 20 % pour l'essai C22 et de 12,4 % à 10,2 % pour l'essai C24). Ceci semble indiquer que pour un procédé continu de ce type la dilution du mélange à purifier ne permet pas de modifier l'extractibilité des impuretés.

Essais C28-C29-C30

On met en œuvre le procédé dans les mêmes conditions que pour l'essai C24 à C27 ci-dessus. Les résultats sont rassemblés dans le tableau 4 ci-après.

(Voir Tableaux 3, 4 (a), 4 (b), 4 (c), pages 8, 9, 10, 11)

Tableau 3

| Essai n° | Pression colonne bar | Pression autoclave bar | Débit $CO_2$ kg/h | Débit alimenta. cc/h | Débit soutirage colonne cc/h | Débit soutirage autoclave distillation cc/h | Alimentation % EtOH | Soutirage % EtOH | Extrait % EtOH | % phase disp |
|---|---|---|---|---|---|---|---|---|---|---|
| C 22 | 80 | 49 | 3,6 | 630 | 580 | 40 | 24,6 | 20,2-19,8 / 20,0-20,4 | 72 | 20 |
| C 23 | 100 | 40 | 2 | 630 | 610 | 8 | 24,6 | 22,4-22,8 / 23,7-23,3 | 52 | 3 / fin essai |
| C 24 | 100 | 49 | 4,5 | 620 | 595 | 15 | 12,4 | 10,6-9,7 / 10 -10,2 | 61 | 9 |
| C 25 | 100 | 50 | 6 - 6,5 | 620 | 570 | 40 | 12,4 | 8,9 - 8,0 / 8,7 - 9 | 43 | 35 |
| C 26 | 90 | 49 | 4,3 | 250 | 215 | 8 | 12,2 | 7,1 - 8,5 / 7,5 | 62 | 21 |
| C 27 | 90 | 49 | 7,4 | 240 | 210 | 18 | 12,2 | 6,1 - 5,6 | 58 | ND |
| C 28 | 90 | 49 | 4,5 | 630 | 610 | 5 | 6 | 4,8 - 5,5 / 5,4 | ∼15 | ND |
| C 29 | 80 | 48 | 7,6 | 630 | 595 | 33 | 6 | 4,9 - 5,1 / 5,8 | ∼18 | 58 / engorgement |
| C 30 | 80 | 48 | 4,5 | 250 | 230 | 4 | 6 | 4,1 - 4,6 / 4,8 | 15 | 28 |
| C 31 | 82 | 49 | 7,8 | 250 | 230 | 15 | 6 | 4,5 - 4,4 / 4,5 | 20 | 37 |

Tableau 4 (a)

| :Temps d'élution : | Dénomination : | Alimentation: | Soutirage: | Soutirage: | Soutirage: | Soutirage: |
|---|---|---|---|---|---|---|
| moyen (en s) : | impureté | non diluée : | colonne : | colonne : | autoclave: | autoclave: |
| : | | C 22 – 23 : | C 22-R 4: | C 23 – R3: | C 22-E 4 : | C23 – E 3: |
| : | | | | | Extrait : | |
| (1): | (2): | (3): | (4) | (5) | (6): | (7): |
| 356 : | Ether : | 5 230 : | 27 : | 134 : | 23 530 : | 53 202 : |
| 516 : | Acétaldéhyde : | 123 : | 0 : | 0 : | 1 643 : | 1 740 : |
| 854 : | Acétone : | 71 : | 0 : | 0 : | 5 086 : | 7 477 : |
| 1 143 : | MEC : | 1 3.4 : | 0 : | 33 : | 21 114 : | 17 385 : |
| 1 252 : | i-PrOH : | 67 : | 10 : | 23 : | 139 : | 438 : |
| 1 719 : | DEC : | 950 : | 0 : | 0 : | 14 434 : | 12 925 : |
| 1 882 : | INI : | 138ll : | 0 : | 0 : ) | 4 133 : ) | 6 078 : |
| 1 926 : | INI : | 35 : | 0 : | 0 : ( | ( | |
| 1 967 : | S-BuOH : | 3 996 : | 613 : | 2 180 : | 54 751 : | 76 026 : |
| 2 014 : | $M_2B_2$ : | 3 389 : | 0 : | 134 : | 49 200 : | 51 643 : |
| 2 404 : | i-BuOH : | 165 : | 0 : | 0 : | 2 021 : | 4 974 : |
| 2 450 : | $P_2$ et $P_3$ : | 97 : | 0 : | 0 : | 2 509 : | 6 024 : |
| 2 691 : | n-BuOH : | 556 : | 73 : | 295 : | 8 820 : | 16 667 : |
| 2 784 : | INI : | 68 : | 0 : | 0 : | 1 258 : | 3 265 : |
| 2 933 : | INI : | 162 : | 0 : | 0 : | 2 808 : | 6 210 : |
| 3 047 : | INI : | 152 : | 0 : | 0 : | 2 087 : | 4 137 : |
| 3 220 : | INI : | 700 : | 0 : | 103 : | 10 865 : | 38 620 : |
| 3 549 : | INI : | 159 : | 0 : | 0 : | 2 120 : | 8 421 : |
| Teneur OH : % en poids : | | 24,6 : | 20,0 : | 23,7 : | 72 : | 52 : |

0 129 459

Tableau 4 (b)

| Temps d'élution moyen (en s) (1) | Dénomination impureté (2) | Alimentation diluée 2 fois | | Soutirage colonne | Soutirage colonne | Soutirage colonne | Soutirage colonne | Soutirage autoclave | Soutirage autoclave | Soutirage autoclave | Soutirage autoclave |
|---|---|---|---|---|---|---|---|---|---|---|---|
| | | Essai C24-C25 (8) | Essai C26-C27 (9) | C24- R 4 (10) | C25-R 4 (11) | C 26- R 3 (12) | C 27 - R3 (13) | C 24- E 4 Extrait (14) | C 25 - E4 Extrait (15) | C 26- E 3 Extrait (16) | C 27-E 3 Extrait (17) |
| 356 | Ether | 2 262 | 1 932 | 55 | 35 | 12 | 13 | 17 256 | 17 260 | 702 | 435 |
| 516 | Acétaldéhyde | 42 | 34 | 0 | 0 | 0 | 0 | 810 | 849 | | |
| 854 | Acétone | 42 | 37 | 0 | 0 | 0 | 0 | 2 843 | 8 992 | 3 297 | |
| 1143 | MEC | 657 | 612 | 0 | 0 | 0 | 0 | 12 903 | 4 490 | 4 034 | 1 932 |
| 1252 | i-PrOH | 47 | 40 | 14 | 10 | 0 | 0 | 214 | 96 | 80 | 88 |
| 1719 | DEC | 507 | 452 | 0 | 0 | 0 | 0 | 10 997 | 3 161 | 4 086 | 2 168 |
| 1882 | INI | 57 | 51 | 0 | 0 | 0 | 0 | 3 777 | 1 038 | 2 188 | 1 207 |
| 1926 | INI | 12 | 2 | 0 | 0 | 0 | 0 | 429 | 177 | 281 | 237 |
| 1967 | S-BuOH | 2 153 | 2 055 | 387 | 350 | 243 | 197 | 61 236 | 19 439 | 42 812 | 23 915 |
| 2014 | $M_2B_2$ | 1 752 | 1 563 | 0 | 0 | 0 | 0 | 45 893 | 12 854 | 19 559 | 11 900 |
| 2404 | i-BuOH | 78 | 55 | 0 | 0 | 0 | 0 | 3 218 | 875 | 2 099 | 1 081 |
| 2450 | $P_2$ et $P_3$ | 41 | 30 | 0 | 0 | 0 | 0 | 3 072 | 648 | 1 888 | 919 |
| 2691 | n-BuOH | 321 | 299 | 42 | 20 | 0 | 0 | 11 550 | 3 442 | 8 922 | 4 383 |
| 2784 | INI | 44 | 37 | 0 | 0 | 0 | 0 | 1 799 | 376 | 1 055 | 528 |
| 2983 | INI | 86 | 70 | 0 | 0 | 0 | 0 | 4 156 | 857 | 2 071 | 1 132 |
| 3047 | INI | 98 | 81 | 0 | 0 | 0 | 0 | 2 703 | 764 | 1 722 | 830 |
| 3220 | INI | 470 | 342 | 0 | 0 | 0 | 0 | 16 590 | 4 590 | 13 212 | 5 884 |
| 3549 | INI | 121 | 82 | 0 | 0 | 0 | 0 | 3 543 | 922 | 2 709 | 1 241 |
| OH % | | 12,4 | | 10,2 | 9,0 | 8,5 | 6,1 | 61,7 | 42,3 | 62,1 | 57,8 |

0 129 459

## Tableau 4 (c)

| Temps d'élution moyen (en s) (1) | Dénomination impureté (2) | Alimentation F605 diluée 4 fois (18) | Soutirage C 28 – R 3 (19) | Soutirage C 29-R 3 (20) | Soutirage C 30- R 4 (21) |
|---|---|---|---|---|---|
| 356 | :Ether | 1 049 | 21 | 30 | 11 |
| 516 | :Acétaldéhyde | | 0 | 0 | 0 |
| 854 | :Acétone | | 0 | 0 | 0 |
| 1 143 | :MEC | 233 | 0 | 0 | 0 |
| 1 252 | :i-PrOH | 27 | 0 | 0 | 0 |
| 1 719 | :DEC | 227 | 0 | 0 | 0 |
| 1 882 | :INI | 27 | 0 | 0 | 0 |
| 1 926 | :INI | | | | 0 |
| 1 967 | :S-BuOH | 1 069 | 413 | 487 | 319 |
| 2 014 | :$M_2B_2$ | 780 | 0 | | 0 |
| 2 404 | :i-BuOH | 27 | 0 | 0 | 0 |
| 2 450 | :$P_2$ et $P_3$ | 20 | 0 | 0 | 0 |
| 2 691 | :n-BuOH | 160 | 44 | 56 | 0 |
| 2 784 | :INI | 20 | 0 | 0 | 0 |
| 2 933 | :INI | 40 | 0 | 0 | 0 |
| 3 047 | :INI | 46 | 0 | 0 | 0 |
| 3 220 | :INI | 187 | 0 | 0 | 0 |
| 3 549 | :INI | | | | |
| % OH | | 6,0 | 5,5 | 4,9 | 4,6 |

0 129 459

**0 129 459**

### Revendications

1. Procédé de purification d'un mélange eau-alcool en $C_1$-$C_2$ issu d'un procédé de fabrication industriel d'alcool en $C_1$-$C_2$ par synthèse chimique, caractérisé en ce qu'on met en contact au moins le mélange à traiter avec au moins un gaz non toxique à l'état liquide ou supercritique et on sépare l'extrait obtenu contenant les impuretés du raffinat purifié.

2. Procédé selon la revendication 1, caractérisé en ce que le mélange eau-alcool en $C_1$-$C_2$-impuretés à purifier provient d'un mélange alcool en $C_1$-$C_2$-impuretés auquel on a rajouté une quantité appropriée d'eau.

3. Procédé selon la revendication 1 ou 2, caractérisé en ce qu'il est mis en œuvre de façon continue ou discontinue.

4. Procédé selon l'une quelconque des revendications 1 à 3, caractérisé en ce que le gaz utilisé est du $CO_2$.

5. Procédé selon la revendication 4, caractérisé en ce qu'on utilise du $CO_2$ liquide à une température comprise entre 0 et 31 °C et à une pression comprise entre 35 et 300 bars.

6. Procédé selon la revendication 4, caractérisé en ce que la température est comprise entre 20 et 30 °C et la pression entre 45 et 150 bars.

7. Procédé selon la revendication 4, caractérisé en ce qu'on utilise du $CO_2$ supercritique à une température supérieure à 35 °C et à une pression comprise entre 73 et 350 bars.

8. Procédé selon l'une quelconque des revendications 4 à 7, caractérisé en ce qu'il est mis en œuvre en continu et en ce qu'on utilise un rapport de débit massique de $CO_2$ liquide ou supercritique au débit massique du mélange à traiter compris entre 1 et 100, de préférence entre 2 et 10.

9. Procédé selon l'une quelconque des revendications 4 à 7, caractérisé en ce qu'il est mis en œuvre en discontinu et en ce qu'on utilise entre 1 et 100 kg de $CO_2$ liquide ou supercritique, de préférence entre 2 et 10 kg par kg de mélange.

10. Procédé selon la revendication 8, caractérisé en ce qu'on effectue l'étape de mise en contact par extraction à contre-courant dans une colonne d'extraction, on injecte le mélange à purifier en tête de colonne d'extraction, on alimente le pied de colonne en $CO_2$ liquide ou supercritique, on soutire en continu le mélange purifié en pied de colonne, on évacue le $CO_2$ contenant les impuretés et un peu d'alcool en $C_1$-$C_2$ en tête de colonne, on fractionne cette même phase dans un dispositif sous pression, on récupère les impuretés sous forme d'un extrait eau-alcool en $C_1$-$C_2$ dégazé et on recycle le $CO_2$ vers la colonne d'extraction après l'avoir ramené aux conditions d'extraction.

11. Procédé selon l'une quelconque des revendications 4 à 9, caractérisé en ce qu'on fractionne en outre l'extrait pour séparer le $CO_2$ des impuretés en solution alcoolique et on recycle le $CO_2$ après l'avoir ramené aux conditions d'extraction à l'étape d'extraction.

12. Procédé selon la revendication 11, caractérisé en ce que ladite séparation est une simple distillation dans le cas où on utilise du $CO_2$ liquide.

13. Procédé selon la revendication 11, caractérisé en ce que :
— On met en contact un mélange eau-alcool en $C_1$-$C_2$-impuretés au moins une fois avec du $CO_2$ liquide en vue de former d'une part une phase $CO_2$ liquide contenant la plus grande partie des impuretés et un peu d'alcool en $C_1$-$C_2$ et, d'autre part un raffinat purifié contenant la presque totalité de l'alcool en $C_1$-$C_2$ engagé et de faibles quantités de $CO_2$.
— On sépare la phase $CO_2$ liquide du raffinat purifié.
— On récupère le raffinat ainsi produit et on le soumet à une opération de dégazage permettant d'éliminer le $CO_2$ liquide.
— On distille la phase $CO_2$ liquide pour obtenir une phase gazeuse de $CO_2$ purifié et une phase liquide constituée essentiellement des impuretés et d'un peu d'alcool en $C_1$-$C_2$, appelée extrait eau-alcool en $C_1$-$C_2$ dégazé.
— On condense la phase gazeuse $CO_2$ que l'on recycle à l'étape initiale de mise en contact.

14. Procédé selon une quelconque des revendications 10 à 13, caractérisé en ce que sur l'extrait eau-alcool en $C_1$-$C_2$ dégazé après élimination du $CO_2$, on effectue une opération de relargage par dilution à l'eau, on décante, on élimine la phase organique et on recycle la phase aqueuse contenant l'alcool en $C_1$-$C_2$ à l'étape d'extraction.

15. Procédé selon l'une quelconque des revendications précédentes caractérisé en ce que le mélange eau-alcool en $C_1$-$C_2$-impuretés à purifier est issu du procédé de synthèse de l'éthanol par hydratation de l'éthylène.

### Claims

1. Process for the purification of a mixture of water/$C_1$-$C_2$-alcohol resulting from an industrial process for the manufacture of $C_1$-$C_2$-alcohol by chemical synthesis characterised in that the mixture to be treated is brought into contact, at least once, with at least one non-toxic gas in the liquid or supercritical state and the extract obtained, containing the impurities, is separated from the purified raffinate.

12

2. Process according to Claim 1, characterised in that the mixture of water/$C_1$-$C_2$-alcohol/impurities which is to be purified results from a mixture of $C_1$-$C_2$-alcohol/impurities to which an appropriate amount of water has been added.

3. Process according to Claim 1 or 2, characterised in that it is carried out continuously or discontinuously.

4. Process according to any one of Claims 1 to 3, characterised in that the gas used is $CO_2$.

5. Process according to Claim 4, characterised in that liquid $CO_2$ at a temperature between 0 and 31 °C and a pressure of between 35 and 300 bars is used.

6. Process according to Claim 4, characterised in that the temperature is between 20 and 30 °C and the pressure between 45 and 150 bars.

7. Process according to Claim 4, characterised in that super-critical $CO_2$ at a temperature above 35 °C and a pressure of between 73 and 350 bars is used.

8. Process according to any one of Claims 4 to 7, characterised in that it is carried out continuously and in that a ratio of the gravimetric flow rate of liquid or super-critical $CO_2$ to the gravimetric flow rate of the mixture to be treated of between 1 and 100, preferably between 2 and 10, is used.

9. Process according to any one of Claims 4 to 7, characterised in that it is carried out discontinuously and in that between 1 and 100 kg of liquid or super-critical $CO_2$, preferably between 2 and 10 kg, are used per kg of mixture.

10. Process according to Claim 8, characterised in that the contacting step is effected by countercurrent extraction in an extraction column, the mixture to be purified is injected at the top of the extraction column, liquid or super-critical $CO_2$ is fed to the bottom of the column, the purified mixture is withdrawn continuously at the bottom of the column, the $CO_2$ containing the impurities and a small amount of $C_1$-$C_2$-alcohol is discharged at the top of the column, this same phase is fractionated in a pressure apparatus, the impurities are recovered in the form of a degassed water/$C_1$-$C_2$-alcohol extract and the $CO_2$ is recycled to the extraction column after having brought it back to the extraction conditions.

11. Process according to any one of Claims 4 to 9, characterised in that the extract is moreover fractionated to separate the $CO_2$ from the impurities in alcohol solution, and the $CO_2$ is recycled after having brought it back to the extraction conditions of the extraction step.

12. Process according to Claim 11, characterised in that where liquid $CO_2$ is used the said separation is a simple distillation.

13. Process according to Claim 11, characterised in that :
— A mixture of water/$C_1$-$C_2$-alcohol/impurities is brought into contact at least once with liquid $CO_2$ so as to form, on the one hand, a liquid $CO_2$ phase containing the greatest part of the impurities and a small amount of $C_1$-$C_2$-alcohol and, on the other hand, a purified raffinate containing virtually all the $C_1$-$C_2$-alcohol employed and small amounts of $CO_2$.
— The liquid $CO_2$ phase is separated from the purified raffinate.
— The raffinate thus produced is recovered and subjected to a degassing operation which allows the liquid $CO_2$ to be removed.
— The liquid $CO_2$ phase is distilled to give a gaseous phase of purified $CO_2$ and a liquid phase consisting essentially of the impurities and a small amount of $C_1$-$C_2$-alcohol, this liquid phase being referred to as a degassed water/$C_1$-$C_2$-alcohol extract.
— The gaseous $CO_2$ phase is condensed and recycled to the initial contacting step.

14. Process according to any one of Claims 10 to 13, characterised in that the degassed water/$C_1$-$C_2$-alcohol extract obtained after removal of $CO_2$ is subjected to a separating-out operation by dilution with water, the mixture is allowed to settle out, the organic phase is removed and the aqueous phase containing the $C_1$-$C_2$-alcohol is recycled to the extraction step.

15. Process according to any one of the preceding claims, characterised in that the water/$C_1$-$C_2$-alcohol/impurities mixture which is to be purified has resulted from the process of synthesising ethanol by hydration of ethylene.

**Patentansprüche**

1. Verfahren zum Reinigen eines Gemisches aus Wasser und $C_1$-$C_2$-Alkohol, das bei einem Verfahren der gewerblichen Herstellung von $C_1$-$C_2$-Alkohol durch chemische Synthese anfällt, dadurch gekennzeichnet, daß man das Gemisch, das behandelt werden soll, mindestens einmal mit mindestens einem nichttoxischen Gas im flüssigen oder super-kritischen Zustand in Berührung bringt und den erhaltenen Extrakt, der die Begleitstoffe enthält, vom gereinigten Raffinat abtrennt.

2. Verfahren nach Anspruch 1, dadurch gekennzeichnet, daß das Gemisch aus Wasser, $C_1$-$C_2$-Alkohol und Begleitstoffen, das gereinigt werden soll, von einem Gemisch aus $C_1$-$C_2$-Alkohol und Begleitstoffen herstammt, dem eine geeignete Menge Wasser zugesetzt worden ist.

3. Verfahren nach Anspruch 1 oder, dadurch gekennzeichnet, daß es kontinuierlich oder diskontinuierlich ausgeführt wird.

4. Verfahren nach einem der Ansprüche 1 bis 3, dadurch gekennzeichnet, daß das verwendete Gas $CO_2$ ist.

13

5. Verfahren nach Anspruch 4, dadurch gekennzeichnet, daß man flüssiges $CO_2$ bei einer Temperatur von 0 bis 31 °C und einem Druck von 35 bis 300 bar verwendet.

6. Verfahren nach Anspruch 4, dadurch gekennzeichnet, daß die Temperatur 20 bis 30 °C und der Druck 45 bis 150 bar beträgt.

7. Verfahren nach Anspruch 4, dadurch gekennzeichnet, daß man super-kritisches $CO_2$ bei einer Temperatur oberhalb 35 °C und bei einem Druck von 73 bis 350 bar verwendet.

8. Verfahren nach einem der Ansprüche 4 bis 7, dadurch gekennzeichnet, daß es kontinuierlich ausgeführt wird und daß man ein Verhältnis von Massendurchsatz an flüssigem oder super-kritischem $CO_2$ zu Massendurchsatz des Gemisches, das behandelt werden soll, von 1 bis 100, vorzugsweise 2 bis 10 anwendet.

9. Verfahren nach einem der Ansprüche 4 bis 7, dadurch gekennzeichnet, daß es diskontinuierlich ausgeführt wird und daß man 1 bis 100 kg, vorzugsweise 2 bis 10 kg flüssiges oder super-kritisches $CO_2$ je kg Gemisch einsetzt.

10. Verfahren nach Anspruch 8, dadurch gekennzeichnet, daß man die Stufe des Inberührungbringens miteinander durch Gegenstromextraktion in einer Extraktionskolonne durchführt, das Gemisch, das gereinigt werden soll, am Kopf der Extraktionskolonne injiziert, am Kolonnenboden flüssiges oder super-kritisches $CO_2$ zuführt, kontinuierlich das gereinigte Gemisch am Kolonnenboden abzieht, das $CO_2$, das die Begleitstoffe und etwas $C_1$-$C_2$-Alkohol enthält, am Kolonnenkopf abzieht, diese Phase in einer Vorrichtung unter Druck fraktioniert, die Begleitstoffe in Form eines entgasten Extraktes aus Wasser und $C_1$-$C_2$-Alkohol isoliert und das $CO_2$ nach Wiedereinstellen der Extraktionsbedingungen in die Extraktionskolonne zurückführt.

11. Verfahren nach einem der Ansprüche 4 bis 9, dadurch gekennzeichnet, daß man außerdem den Extrakt fraktioniert, um das $CO_2$ von den Begleitstoffen in alkoholischer Lösung abzutrennen, und das $CO_2$ in die Extraktionsstufe zurückführt, nachdem es auf die Extraktionsbedingungen zurückgebracht worden ist.

12. Verfahren nach Anspruch 11, dadurch gekennzeichnet, daß die Trennung eine einfache Destillation ist, wenn man flüssiges $CO_2$ verwendet.

13. Verfahren nach Anspruch 11, dadurch gekennzeichnet, daß man :
— ein Gemisch aus Wasser, $C_1$-$C_2$-Alkohol und Begleitstoffen mindestens einmal mit flüssigem $CO_2$ zusammenbringt, um einerseits eine Phase aus flüssigem $CO_2$ zu bilden, die den überwiegenden Anteil der Begleitstoffe und etwas $C_1$-$C_2$-Alkohol enthält und andererseits ein gereinigtes Raffinat, das fast den gesamten eingesetzten $C_1$-$C_2$-Alkohol und geringe Mengen an $CO_2$ enthält,
— die $CO_2$-Flüssigphase vom gereinigten Raffinat trennt,
— das so erzeugte Raffinat gewinnt und einem Entgasungs-Arbeitsgang unterwirft, der es ermöglicht, das flüssige $CO_2$ zu entfernen,
— die $CO_2$-Flüssigphase destilliert, um eine gereinigte $CO_2$-Gasphase und eine Flüssigphase zu erhalten, die im wesentlichen aus den Begleitstoffen und etwas $C-1$-$C_2$-Alkohol besteht und als entgaster Wasser-$C_1$-$C_2$-Alkohol-Extrakt bezeichnet wird,
— die $CO_2$-Gasphase kondensiert und in die Anfangsstufe des Zusammenbringens zurückführt.

14. Verfahren nach einem der Ansprüche 10 bis 13, dadurch gekennzeichnet, daß man den nach dem Abtrennen des $CO_2$ entgasten Wasser-$C_1$-$C_2$-Alkohol-Extrakt durch Verdünnen mit Wasser ausschüttelt, dekantiert, die organische Phase entfernt und die wäßrige Phase, die den $C_1$-$C_2$-Alkohol enthält, in die Extraktionsstufe zurückführt.

15. Verfahren nach einem der vorangehenden Ansprüche, dadurch gekennzeichnet, daß das Gemisch aus Wasser, $C_1$-$C_2$-Alkohol und Begleitstoffen, das gereinigt werden soll, bei dem Verfahren der Synthese von Ethanol durch Hydratation von Ethylen angefallen ist.

0 129 459

FIG.1

## FIG. 2

## FIG. 3